# EUROPEAN PATENT APPLICATION

(11) **EP 2 924 123 A1**
(43) Date of publication of application: **30.09.2015**
(21) Application number: 13857642.6
(22) Date of filing: 20.09.2013
(51) Int. Cl.: C12P 7/18, C12N 1/19, C12N 1/21, C12N 15/09

(54) **1,4-BUTANEDIOL MANUFACTURING METHOD AND MICROORGANISM**

(30) Priority: 26.11.2012 JP 2012257882
(71) Applicant: Showa Denko K.K., Tokyo 105-8518 (JP)
(72) Inventor: AOKI, Hirobumi, Tokyo 105-8518 (JP); KOKIDO, Yuzuru, Tokyo 105-8518 (JP); HASHIMOTO, Yoko, Tokyo 105-8518 (JP); YONEDA, Tadashi, Tokyo 105-8518 (JP)
(74) Representative: Strehl Schübel-Hopf & Partner
(86) International application number: PCT/JP2013/075513
(87) International publication number: WO 2014/080687

(57) **Abstract**

A method of manufacturing 1,4-butanediol, using a microbe and/or a culture thereof, by an enzyme reaction system that uses acyl-CoA reductase, via 3-hydroxybutyryl-CoA, crotonyl-CoA and 4-hydroxybutyryl CoA in this order, wherein the reactivity of the acyl-CoA reductase to 4-hydroxybutyryl CoA is greater than or equal to 0.05 times of the reactivity of the acyl-CoA reductase to 3-hydroxybutyryl-CoA.

## Description

### BACKGROUND OF THE INVENTION

### 1. Field of the Invention

The present invention relates to a method of manufacturing 1,4-butanediol and a microbe.

### 2. Description of the Related Art

In recent years, attention is paid to a compound manufacturing process with a renewable source as a raw material from the viewpoint of depletion of fossil resource, a countermeasure for global warming, or the like. In particular, a so-called bio-refinery has widely been studied wherein a variety of compounds as raw materials for a polymer or compounds as raw materials for a chemical product are manufactured in biochemical processes with biomass as raw materials.

For compounds that are expected as raw material conversion of biomass, 1,4-butanediol may be used. 1,4-butanediol is widely used as a synthetic raw material for a fine organic chemical product, a monomer unit of a polyester and an engineering plastic or the like, and its market scale is large. Thus, demand for a method of efficiently manufacturing 1,4-butanediol is becoming large in a biochemical process with a renewable source such as biomass or the like as a raw material.

For methods of manufacturing 1,4-butanediol using a biochemical process, for example, methods disclosed in Patent documents 1 and 2 and Non-patent document 1 may be used.
[Patent document 1] Japanese Patent No. 4380704 specification
[Patent document 2] International Publication No. 2008/115840 official gazette

[Non-patent document 1] Harry Yim et al., Metabolic engineering of Escherichia coli for direct production of 1,4-butanediol, Nature Chemical Biology, 7, 445-452 (2011).

### SUMMARY OF THE INVENTION

However, the methods disclosed in Patent documents 1 and 2 and Non-patent document 1 are complicated processes.

For a problem as described above, there is provided a new method of manufacturing 1,4-butanediol capable of economically obtaining 1,4-butanediol.

The present invention includes the following.
[1] A method of manufacturing 1,4-butanediol, using a microbe and/or a culture thereof, by an enzyme reaction system that uses acyl-CoA reductase, via 3-hydroxybutyryl-CoA, crotonyl-CoA and 4-hydroxybutyryl CoA in this order,
   wherein the reactivity of the acyl-CoA reductase to 4-hydroxybutyryl CoA is greater than or equal to 0.05 times of the reactivity of the acyl-CoA reductase to 3-hydroxybutyryl-CoA.
[2] The method of manufacturing 1,4-butanediol according to [1],
   wherein the acyl-CoA reductase is aldehyde dehydrogenase (acylation)(EC number: 1.2.1.10).
[3] The method of manufacturing 1,4-butanediol according to [1],
   wherein the microbe includes a gene that codes the acyl-CoA reductase and is any one of undermentioned genes (a) to (c):
   (a) a gene that has a base sequence of sequence number 6;
   (b) a gene that has a base sequence such that one or more bases are deleted, substituted, or added in a base sequence of sequence number 6, wherein the gene has a base sequence with an identity greater than or equal to 90% with respect to the base sequence of sequence number 6;
   (c) a gene that hybridizes with a gene that has a base sequence complementary with a gene that has a base sequence described in sequence number 6 on a stringent condition.
[4] The method of manufacturing 1,4-butanediol according to [1],
   wherein the microbe is Escherichia coli, a yeast, a corynebacterium or a clostridial bacteria.
[5] A microbe that manufactures 1,4-butanediol by an enzyme reaction system that uses acyl-CoA reductase, via 3-hydroxybutyryl-CoA, crotonyl-CoA and 4-hydroxybutyryl CoA in this order,
   wherein the reactivity of the acyl-CoA reductase to 4-hydroxybutyryl CoA is greater than or equal to 0.05 times of the reactivity of the acyl-CoA reductase to 3-hydroxybutyryl-CoA.
[6] The microbe according to [5],
   wherein the microbe is Escherichia coli, a yeast, a corynebacterium or a clostridial bacteria.

A method of economically manufacturing 1,4-butanediol is provided.

### BRIEF DESCRIPTION OF THE DRAWINGS

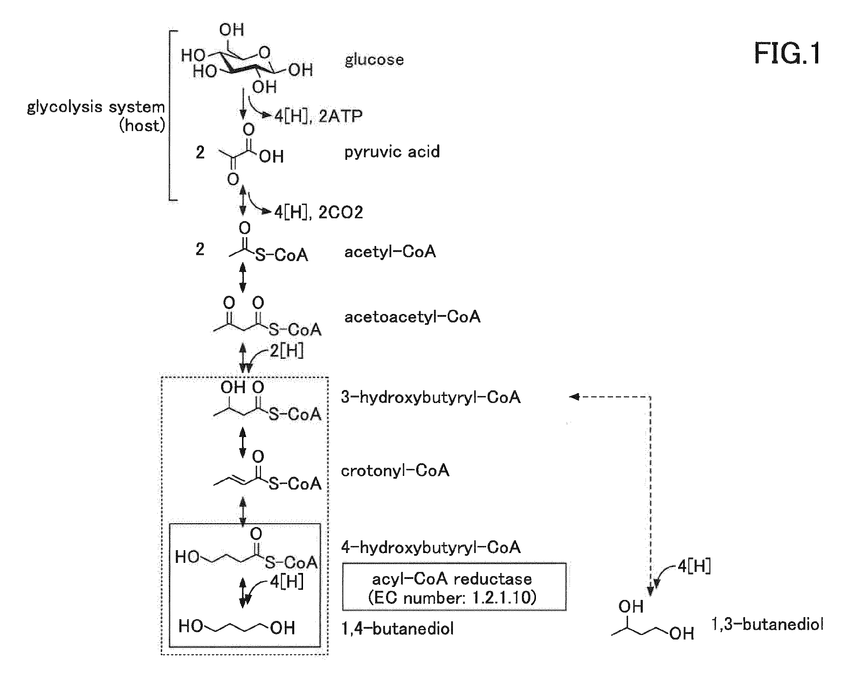

Fig. 1 is a view illustrating an example of an enzyme system of a method of manufacturing 1,4-butanediol of an embodiment.

### DETAILED DESCRIPTION OF THE PREFERRED EMBODIMENTS

The present invention will be described in detail below. Here, in this specification, "CoA" means "coenzyme A". Furthermore, "%" indicates "% by mass" unless otherwise described. "ppm" is mass basis.

In this embodiment, an enzyme reaction system is used in which 1,4-butanediol is manufactured, using a microbe, via acetoacetyl-CoA, 3-hydroxybutyryl-CoA, crotonyl-CoA and 4-hydroxybutyryl CoA in this order. Here, one of features of the present invention is a method of using a microbe or a culture thereof capable of selectively manufacturing 1,4-butanediol with high productivity.

After diligently studying means to solve the above described problem, the present inventors have found that a method of manufacturing 1,4-butanediol with a higher reactive selectivity to 4-hydroxybutyryl CoA can be provided by appropriately selecting a gene that codes acyl-CoA reductase in a method in which 1,4-butanediol is manufactured via crotonyl-CoA and 4-hydroxybutyryl CoA in this order from 3-hydroxybutyryl-CoA as a substrate (or may be an intermediate or a precursor) by an enzyme reaction system using a microbe. As a result, it is found that generation of 1,3-butanediol from 3-hydroxybutyryl-CoA is suppressed and the productivity of 1,4-butanediol is improved.

Further, in a preferred embodiment of the invention, by appropriately selecting a gene that codes acyl-CoA reductase, a method of manufacturing 1,4-butanediol can be provided in which the reactivity to 4-hydroxybutyryl CoA is at least greater than or equal to 0.05 times of the reactivity to 3-hydroxybutyryl-CoA.

Hereinafter, a feature of a microbe, a method of manufacturing the microbe, a method of using the microbe (in other words, a method of manufacturing 1,4-butanediol), a method of obtaining the manufactured 1,4-butanediol or the like, that is used in this embodiment, is explained.

### (Host microbe)

The host microbe used in this embodiment is not particularly limited as long as various genes, which will be explained later, can be introduced therein and a gene recombination technique can be applied to the host microbe.

More specifically, the host microbe is one capable of being transformed into a microbe that can manufacture 1,4-butanediol using 3-hydroxybutyryl-CoA as a substrate, via crotonyl-CoA and 4-hydroxybutyryl-CoA in this order under an appropriate culturing condition, which will be explained later.

From the viewpoint of industrial availability, for a specific example, Escherichia coli, a yeast, a coryneform bacteria or a clostridial bacteria may be used. For a yeast, Saccharomyces cerevisiae, Schizosaccharomyces pombe, Kluyveromyces lactis, Kluyveromyces marxianus or the like may be used. For a coryneform bacteria, Corynebacterium glutamicum, Corynebacterium efficiens, Brevibacterium divaricatum, Brevibacterium saccharolyticum, Brevibacterium immariophilum, Brevibacterium lactofermentum, Brevibacterium roseum, Brevibacterium flavum, Brevibacterium thiogenitalis, Corynebacterium acetoacidophilum, Corynebacterium acetoglutamicum, Corynebacterium callunae, Corynebacterium lilium, Corynebacterium mellassecola, Microbacterium ammoniaphilum or the like may be used. For a clostridial bacteria, Clostridium kluyveri, Clostridium acetobutylicum, Clostridium aminobutyricum, Clostridium beijerinckii, Clostridium saccharoperbutylacetonicum or the like may be used. Among these, it is preferable to use Escherichia coli, Saccharomyces cerevisiae, Schizosaccharomyces pombe or Corynebacterium glutamicum, because transformation thereof is easy, and it is more preferable to use Escherichia coli.

Further, the transformation microbe of the embodiment may be used in various embodiments including a cultured microbial cell itself and a culture thereof. The culture of the microbe of the embodiment includes, specifically, a suspended material of a cultured microbial cell by a medium such as a culture medium, buffer or the like, a cell-free extract from a cultured microbial cell, and further a processed material such as a material obtained by condensing, purifying or extracting a component that catalyzes the reaction from the cell-free extract or the like. The culture of the microbe of the embodiment further includes a material in which a processed material of the microbe is immobilized on an insoluble carrier. For such an immobilized carrier, a compound that forms a water-insoluble solid content containing the above described microbial cell or its processed material such as polyacrylamide, polyvinyl alcohol, poly(N-vinylformamide), polyallylamine, polyethyleneimine, methylcellulose, glucomannan, alginate, carrageenan or the like, further, a copolymer or a crosslinked material thereof or the like may be used. A kind of these may be used or a mixture of two or more kinds of these may be used. Further, a material in which the microbe or its extracting solution or extracting component is held on an object previously formed as a solid material such as an activated carbon, porous ceramics, a glass fiber, a porous polymer plastic body, a nitrocellulose film or the like may be used as the culture of the microbe as well.

### (Transformation microbe)

The transformation microbe used in this embodiment has at least each enzyme system of an enzyme reaction system by which 1,4-butanediol can be manufactured, using 3-hydroxybutyryl-CoA as a substitute, via crotonyl-CoA and 4-hydroxybutyryl-CoA in this order, in addition to an enzyme system that is originally possessed by the host microbe. Hereinafter, a gene that codes each enzyme system is explained.

### (Gene that codes enoyl-CoA hydratase)

A gene that codes enoyl-CoA hydratase used in this embodiment is not particularly limited as long as it is a gene that codes an enzyme capable of catalyzing a reaction in which 3-hydroxybutyryl-CoA is dehydrated as a substrate to generate crotonyl-CoA.

For a specific example of the gene that codes the enzyme capable of catalyzing the reaction in which crotonyl-CoA is generated from (S)-3-hydroxybutyryl-CoA, used in this embodiment, although not particularly limited, a gene or a homolog thereof that codes enoyl-CoA hydratase (EC number: 4.2.1.17) may be used. For the detail of the above mentioned enzyme, a Non-patent document such as "Moskowitz, G.J. and Merrick, J.M., Metabolism of poly-β-hydroxybutyrate. II. Enzymatic synthesis of D-(-)-β-hydroxybutyryl coenzyme A by an enoyl hydrase from Rhodospirillum rubrum, Biochemistry 8 (1969), 2748-2755." or the like may be referred.

Further, for a specific example of the gene that codes the enzyme capable of catalyzing the reaction in which crotonyl-CoA is generated from (R)-3-hydroxybutyryl-CoA, used in this embodiment, although not particularly limited, a gene, a homolog thereof or the like that codes enoyl-CoA hydratase (EC number: 4.2.1.55 (3-hydroxybutyryl-CoA dehydratase) or EC number: 4.2.1.119) may be used. For the detail of the above mentioned enzyme, Non-patent documents such as "Fukui, T., Shiomi, N. and Doi, Y., Expression and characterization of (R)-specific enoyl coenzyme A hydratase involved in polyhydroxyalkanoate biosynthesis by Aeromonas caviae, J. Bacteriol. 180 (1998), 667-673.", "Moskowitz, G.J. and Merrick, J.M., Metabolism of poly-β-hydroxybutyrate. II. Enzymatic synthesis of D-(-)-β-hydroxybutyryl coenzyme A by an enoyl hydrase from Rhodospirillum rubrum, Journal Biochemistry 8 (1969), 2748-2755." or the like may be referred.

### (Gene that codes vinylacetyl-CoA delta-isomerase)

In this embodiment, a gene that codes vinylacetyl-CoA delta-isomerase is not particularly limited as long as it is a gene that codes an enzyme capable of catalyzing a reaction in which vinylacetyl-CoA is generated by transitioning olefin of crotonyl-CoA.

For a specific example of the enzyme capable of catalyzing the above mentioned reaction, although not limited, vinylacetyl-CoA delta-isomerase (EC number: 5.3.3.3), a homolog thereof or the like may be used. For the detail of the above mentioned enzyme, for example, a Non-patent document such as "Fermentation of 4-aminobutyrate by Clostridium aminobutyricum: cloning of two genes involved in the formation and dehydration of 4-hydroxybutyryl-CoA, Archives of Microbiology, 174(3) 189-199 (2000)." or the like may be referred.

### (Gene that codes 4-hydroxybutyryl-CoA dehydratase)

In this embodiment, a gene that codes 4-hydroxybutyryl-CoA dehydratase is not particularly limited as long as it is a gene that codes an enzyme that catalyzes a reaction in which vinylacetyl-CoA is hydrated to generate 4-hydroxybutyryl-CoA.

For a specific example of the above mentioned enzyme capable of catalyzing the reaction, although not limited, 4-hydroxybutyryl-CoA dehydratase (EC number: 4.2.1.120), a homolog thereof or the like may be used. For the detail of the above described enzyme, for example, a Non-patent document such as "Fermentation of 4-aminobutyrate by Clostridium aminobutyricum: cloning of two genes involved in the formation and dehydration of 4-hydroxybutyryl-CoA, Archives of Microbiology, 174(3) 189-199 (2000)." or the like may be referred. Here, an example in this Non-patent document relates to a multienzyme of 4-hydroxybutyryl-CoA dehydratase and the above described vinylacetyl-CoA delta-isomerase (EC number: 5.3.3.3) and a gene that codes thereof. However, as long as functions of each enzyme can be appropriately provided, each enzyme or an individual gene that codes a catalytic subunit protein may be used.

### (Gene that codes acyl-CoA reductase)

In this embodiment, a gene that codes acyl-CoA reductase is not particularly limited as long as it codes an enzyme capable of catalyzing a reaction in which 4-hydroxybutyryl-CoA is reduced and 4-hydroxybutanal is generated and it is 4-hydroxybutyryl CoA selective acyl-CoA reductase. Here, although the obtained 4-hydroxybutanal is led to 1,4-butanediol by alcohol reductase that is normally possessed by the host, which will be explained later, a gene that codes alcohol reductase may be additionally expressed to manufacture 1,4-butanediol.

For a specific example of an enzyme capable of catalyzing the above-mentioned reaction, aldehyde dehydrogenase (acylation)(EC number: 1.2.1.10) or a homolog thereof may be used.

In this embodiment, "4-hydroxybutyryl CoA selective acyl-CoA reductase" is not limited as long as it is an enzyme whose reactivity to 4-hydroxybutyryl CoA is high, however, preferably, it is referred for one whose reactivity to 4-hydroxybutyryl CoA with respect to the reactivity to 3-hydroxybutyryl-CoA is greater than or equal to 0.05, more preferably, greater than or equal to 0.07, and furthermore preferably, greater than or equal to 0.2 times.

Further, substrate specificity of an enzyme may be confirmed by a method in which free CoA, that is generated during a constant period by a reduction reaction using each substrate, is colored by coupling with a color pigment DTNB (5,5'-dithiobis-2-nitrobenzoic acid) and its absorbance is measured or the like. Alternatively, an ordinary method for quantifying CoA that is successively generated in accordance with a progression of a reduction reaction may be used. Further, it can be relatively evaluated that the enzyme that catalyzes the reduction process is a desired 4-hydroxybutyryl CoA selective acyl-CoA reductase by grasping a ratio of yield of each of 1,3-butanediol and 1,4-butanediol, or a ratio of yield of each of 3-hydroxybutanal and 4-hydroxybutanal generated during processes of executing a part of or all of the embodiment by information such as high performance liquid chromatography (HPLC), gas chromatography (GC) or the like.

### (Method of manufacturing transformation microbe)

Introduction of a gene to a host microbe may be performed by appropriately combining various known methods, for example, such as a method based on restriction enzyme / ligation, an In-Fusion cloning method or the like, such that the above described gene or its part is connected to an appropriate vector and the obtained recombinant vector is introduced into the host so that a purposed gene can be expressed. Alternatively, introduction of a gene to a host microbe may be performed by inserting a target gene or its part at an arbitrary site of a genome by a homologous recombination. "Part" indicates a part of each gene capable of expressing a protein coded by the respective gene when being introduced into the host. In this invention, a gene includes DNA and RNA, and is preferably DNA.

For a vector that connects the gene, it is not particularly limited as long as it is duplicable by a host, and for example, a plasmid, a phage, a cosmid or the like that is used for transgenesis in an Escherichia coli may be used. For a plasmid, for example, pHSG398, pUC18, pBR322, pSC101, pUC19, pUC118, pUC119, pACYC117, pBluescript II SK(+), pET17b, pETDuet-1, pACYCDuet-1, pCDFDuet-1, pRSFDuet-1, pCOLADuet-1 or the like may be used. For a phage, for example, λgt10, Charon 4A, EMBL-, M13mp18, M13mp19 or the like may be used. Some of them are commercially available and a commercial item (kit) may be used as it is in accordance with its procedures manual, or after appropriately altering.

In the above described vector, an appropriate expression promoter may be connected at upstream of a gene such that the inserted gene is surely expressed. The expression promoter to be used is not particularly limited and is appropriately selected by a person(s) skilled in the art in accordance with the host. For example, T7promoter, lacpromoter, trppromoter, trcpromoter or λ-PLpromoter that is used for expressing a foreign gene in Escherichia coli, a Nar promoter region of nitrate reductase gene narGHJI operon that relates to nitrate respiration derived from Escherichia coli, or a promoter region of Frd gene that is nitrate reductase gene of Escherichia coli may be used.

Further, for some cases, it is preferable that the original gene of the host microbe is disrupted so that the gene is not expressed. For a method of disrupting the gene, known methods that are used for disrupting a gene in Escherichia coli may be used. Specifically, a method that is used for manufacturing a knockout cell or the like in this technical field such as a method of disrupting a gene (gene targeting method) using a vector (targeting vector) that generates a homologous recombination at an arbitrary position of a target gene, a method (gene trapping method) in which a trap vector (reporter gene that does not have promoter) is inserted at an arbitrary position of a target gene to disrupt the gene and deactivate its function, a method of combination thereof or the like may be used.

The position at which the homologous recombination is generated or the position to which the trap vector is inserted is not particularly limited as long as it is a position at which modification to deactivate the expression of the target gene to be disrupted, but preferably, is a transcriptional control region.

Further, for a method of introducing the vector to the host, although not particularly limited, for example, a method of using calcium ion, a protoplast method, an electroporation method or the like that is generally used for introducing a vector to Escherichia coli may be used.

A method of inserting a target gene to an arbitrary position on a genome by homologous recombination may be performed by inserting the target gene to a homogenous sequence of a sequence on the genome with a promoter and introducing the nucleic acid fragment to a cell by electroporation to generate homologous recombination. When introducing to the genome, a stock may be selected by which homologous recombination easily occurred when using a nucleic acid fragment to which the target gene and a drug-resistant gene are connected. Further, the target gene may be introduced by homologous recombination by inserting a gene to which a drug-resistant gene and a gene that becomes fatal under a specific condition are connected to the genome by the above described method by homologous recombination, and thereafter, substituting the drug-resistant gene and the gene that becomes fatal under a specific condition.

Further, for a method of selecting a recombinant microbe in which the target gene is introduced, although not particularly limited, it is preferably to use a method by which only a recombinant microbe in which the target gene is introduced can be easily selected.

### (Method of using microbe for manufacturing 1,4-butanediol, culturing condition and method of obtaining obtained 1,4-butanediol)

Fig. 1 illustrates an example of an enzyme system of the method of manufacturing 1,4-butanediol of the embodiment. In this embodiment, 1,4-butanediol can be obtained by using a culture in which the above described series of genes are expressed in a microbial body by means of transformation or the like. Here, each of the genes, individually or as a series of cluster, transforms the host microbe by being inserted into an arbitrarily vector. By culturing the obtained transformed body in a medium with an appropriate carbon source, for example, glucose, each of the genes is expressed. For a gene that can be constitutively expressed in a host, the gene can be expressed by culturing the transformed body in the medium. On the other hand, when each of the genes is constituted under a control of a regulator that is provided on a vector, each of the genes that codes is expressed by adding a deriving substrate to transfer to a deriving environment. Here, culturing in this embodiment includes all of culturing conditions for normal microbe culturing, and further, a step of culturing means that culturing with sufficient period and condition for the microbe to manufacture 1,4-butanediol.

Normally, butanediol generated by a combination of general enzymes coded by the gene of the embodiment includes a mixture of 1,3-butanediol and 1,4-butanediol by a simultaneous generation. This means that when enoyl-CoA hydratase and 4-hydroxybutyryl CoA dehydratase are effected under an existence of a precursor (or an intermediate) 3-hydroxybutyryl-CoA, a state of equilibrium between 3-hydroxybutyryl-CoA and 4-hydroxybutyryl CoA is rapidly generated by the effect of them. When acyl-CoA reductase is effected under the state of equilibrium, 3-hydroxybutanal and 4-hydroxybutanal are equilibrium ratio dependently generated by a reduction reaction to aldehydes that is CoA dependent using both acyl-CoAs as substrates. These aldehydes are led to 1,3-butanediol and 1,4-butanediol by host derived alcohol reductase or the like. In this embodiment, by effecting 4-hydroxybutyryl CoA selective acyl-CoA reductase in this reduction process, a yield ratio of 3-hydroxybutanal can be reduced and ratio of 4-hydroxybutanal can be improved in a generation process of 3-hydroxybutanal and 4-hydroxybutanal. As a result, productivity of 1,4-butanediol is improved.

### (Supplying 3-hydroxybutyryl-CoA)

A method of supplying 3-hydroxybutyryl-CoA in the method of manufacturing 1,4-butanediol of the embodiment is not particularly limited, and various known methods may be used.

As an example, first, under a condition in which acetyl-CoA obtained from a known reaction path such as a glycolysis system or the like is supplied, acetoacetyl-CoA can be supplied using an enzyme reaction with a gene or a homolog thereof that codes β-ketothiolase (EC number: 2.3.1.9) by which one molecule of CoA is desorbed from two molecules of acetyl-CoA to supply acetoacetyl-CoA. Alternatively, acetoacetyl-CoA may be supplied using a gene or a homolog thereof that codes acetoacetyl-CoA synthase (EC number: 2.3.1.194) that catalyzes a reaction that irreversibly produces acetoacetyl-CoA using acetyl-CoA and malonyl-CoA as substrates. For the detail of the acetoacetyl-CoA synthase, for example, a Non-patent document such as "Unprecedented acetoacetyl-coenzyme A synthesizing enzyme of the thiolase superfamily involved in the mevalonate pathway., Proc. Natl. Acad. Sci., U. S. A. 107, 11265 - 11270 (2010)" or the like may be referred to.

Next, by an enzyme reaction using acetoacetyl-CoA reductase (EC number: 1.1.1.36), 3-hydroxybutyryl-CoA dehydrogenase (EC number: 1.1.1.35), 3-hydroxyacyl CoA dehydrogenase (EC number: 1.1.1.157) or a homolog thereof that catalyzes a reaction in which the obtained acetoacetyl-CoA is reduced to give 3-hydroxybutyryl-CoA, 3-hydroxybutyryl-CoA can be obtained. Here, when acetoacetyl-CoA reductase (EC number: 1.1.1.36) is used, (R)-3-hydroxybutyryl-CoA can be obtained, and when 3-hydroxybutyryl-CoA dehydrogenase (EC number: 1.1.1.35) or 3-hydroxyacyl CoA dehydrogenase (EC number: 1.1.1.157) is used, (S)-3-hydroxybutyryl-CoA can be obtained.

Further, for an alternative example, a propionic acid CoA transferase (EC number: 2.8.3.1) is known for an enzyme that directly transfers CoA to 3-hydroxybutanoic acid. Under a status in which a gene or its homolog that codes propionic acid CoA transferase is expressed, and acetyl-CoA, that becomes a donor in a CoA transferring reaction, is supplied, 3-hydroxybutyryl-CoA can be generated by supplying 3-hydroxybutanoic acid to a microbe or a culture including the microbe.

A homolog referred to in the embodiment includes an ortholog and a paralog. An ortholog refers to a set of corresponding genes among species generated from a gene of a common ancestor by means of speciation and enzymes obtained from such genes. A paralog refers to a set of corresponding genes among species generated by means of not speciation but gene duplication in an identical species and enzymes obtained from such genes. A homolog refers to genes that have an identity in sequences thereof, regardless of an ortholog or a paralog, and enzymes obtained from such genes.

More specifically, a homolog (gene) in this embodiment is a gene that has a base sequence with an identity greater than or equal to 90%, preferably an identity greater than or equal to 95%, and more preferably, a gene that is completely identical to such a gene or wherein one or several bases thereof are deleted, substituted, or added.

Furthermore, a homolog gene includes a gene that hybridizes with a gene that has a base sequence complementary with a target gene on a stringent condition. Specifically, it is possible to acquire a gene or an enzyme that is obtained by transformation caused by such a gene, by applying a homology retrieval program (for example, BLAST or FASTA) to a publicly-known data base, or based on an ordinary method such as hybridization, polymerase chain reaction (PCR) or the like under a stringent condition that uses a probe that is composed of at least a part of an identified gene (DNA that is composed of a base sequence complementary with DNA that is composed of a base sequence of such a gene). Furthermore, it is possible for a person(s) skilled in the art to execute self-design by substituting a base sequence or the like. Here, for a stringent condition referred herein, for example, a condition for executing hybridization as described in a Non-patent document of "Molecular Cloning - A LABORATORY MANUAL THIRD EDITION (Joseph Sambrook, David W. Russell., Cold Spring Harbor Laboratory Press)". Specifically, a hybridizing condition is to be retained with a probe in a solution that includes 6 x SSC (a composition of 1 x SSC: 0.15 M of sodium chloride, 0.015 M of sodium citrate, pH: 7.0), 0.5% of SDS, 5 x Denhardt's solution and 100 mg/mL of herring sperm DNA, at a constant temperature of 65 °C for 8 - 16 hours.

### (Culturing method)

For example, a reaction in the present invention is most conveniently achieved in such a manner that a transformed body is cultured in a nutritive medium such as an LB medium at a temperature of 15 °C - 40 °C, desirably 18 °C - 37 °C for about 24 hours, subsequently implanted to a medium with a normal carbon source, for example, a carbon source that is 0.01 - 50%, desirably 0.1 - 30%, of glucose, and continuously cultured at a similar temperature for about 1 hour - 200 hours, wherein 1,4-butanediol is stored in a culture solution in such a process. Furthermore, a carbon source may be added continuously or intermittently depending on consumption of a carbon source that is caused by proliferation of a bacteria or proceeding of a reaction, and in such a case, a concentration of a carbon source in a reaction fluid is not limited to one described previously.

For a medium carbon source for culturing a microbe, it is possible to use, for example, a saccharide such as glucose, sucrose, fructose or the like, a polyol such as glycerol or the like, an organic substance such as ethanol, acetic acid, citric acid, succinic acid, lactic acid, benzoic acid, fatty acid or the like, or an alkali metal salt thereof, an aliphatic hydrocarbon such as n-paraffin or the like, an aromatic hydrocarbon or a natural organic substance such as peptone, meat extract, fish extract, soybean flour, bran or the like, for example, singly or in combination thereof, at a concentration of normally 0.01% - 30%, desirably about 0.1% - 20%.

For a medium nitrogen source for culturing a microbe, it is possible to use, for example, an inorganic nitrogen compound such as ammonium sulfate, ammonium phosphate, sodium nitrate, potassium nitrate or the like, a nitrogen-containing organic substance such as urea, uric acid or the like, or a natural organic substance such as peptone, meat extract, fish extract, soybean flour or the like, singly or in combination thereof, at a concentration of normally 0.01% - 20%, desirably about 0.1% - 10%.

Moreover, it is possible to add a phosphate such as potassium dihydrogen phosphate or the like, or a metal salt such as magnesium sulfate, ferrous sulfate, calcium acetate, manganese chloride, copper sulfate, zinc sulfate, cobalt sulfate, nickel sulfate or the like, in accordance with necessity, for growth of a bacteria or improvement of an enzyme activity. A concentration for addition thereof is different depending on a culturing condition, and normally, is 0.01% - 5% for a phosphate, 10 ppm - 1% for a magnesium salt, or about 0.1 ppm - 1,000 ppm for other compounds. Furthermore, for a source of supply for a vitamin, an amino acid, a nucleic acid or the like, it is possible to add, for example, about 1 ppm - 100 ppm of yeast extract, casamino acid, or a yeast nucleic acid, depending on a selected medium, for growth of a bacteria or improvement of an enzyme activity.

It is desirable to adjust a pH of a medium to 4.5 - 9, desirably 5 - 8. Furthermore, in order to reduce an impurity in a reaction fluid and simplify subsequent fractionation of a product, it is useful to fractionate a bacterial body that is preliminarily cultured in a medium as described previously from a culture solution by a method such as centrifugation or membrane filtration, and again suspend and react in water that includes a reaction raw material, physiological saline, a buffer that has a pH adjusted to be comparable to a pH for culturing and composed of phosphoric acid, acetic acid, boric acid, tris(hydroxymethyl)aminomethane or the like and a salt thereof, or the like. Although a pH during a reaction is able to be normally retained in a case where a buffer with a sufficient concentration is used, it is desirable to be adjusted appropriately by using sodium hydroxide, ammonia or the like so as to provide a similar pH in a case where deviation from a pH as described above is caused by proceeding of a reaction.

When a reaction rate is lowered due to 1,4-butanediol stored in a reaction fluid, a method is preferable that adds water, physiological saline, a reaction buffer or the like, into a reaction fluid depending on a concentration of a product to execute continuous dilution thereof. Furthermore, at a point of time when a reaction rate is lowered, a bacteria is fractionated and a supernatant is recovered as a product solution, wherein a fractionated bacteria is again returned to a solution or suspension that includes a reaction raw material and thereby it is possible to recover a reaction rate. It is possible to execute such an operation continuously or even batch-wise by using a centrifuge, a separation film or the like.

It is possible to execute separation, recovery and purification of 1,4-butanediol produced in a reaction fluid, at a point of time when an amount of produced 1,4-butanediol reaches a substantial amount, by using a general means of separation, recovery, and purification of an organic compound, after a bacterial body is eliminated from such a reaction fluid by means of centrifugation or for such a reaction fluid directly. For example, extraction from a filtrate wherein a bacterial body and the others are eliminated from a culture solution is executed by using an appropriate organic solvent. Such an extract is directly distilled away, is further extracted again with an appropriate solvent, purified by using silica gel chromatography or the like, is subjected to multistage distillation or the like, and thereby, 1,4-butanediol is obtained at a high purity.

### (Examples)

The present invention is further explained in detail by explaining examples.

Table 1 illustrates assumed reaction processes, enzymes that catalyze the respective reaction processes, and sequence numbers of used genes that code the respective enzymes. Here, the sequence number illustrated for each of the genes corresponds to the sequence number in a sequence listing.

**[Table 1]**

| SEQUENCE NUMBER | ENZYME TO BE CODED | DERIVATION |
|---|---|---|
| SEQUENCE NUMBER 1 | β-ketothiolase (EC number: 2.3.1.9) | atoB (Escherichia coli K12 stock derived sequence, artificial composition) |
| SEQUENCE NUMBER 2 | 3-hydroxybutyryl-CoA dehydrogenase (EC number: 1.1.1.35) | hbd (Clostridium kluyveri DSM555 derived sequence, artificial composition) |
| SEQUENCE NUMBER 3 | enoyl-CoA hydratase (EC number: 4.2.1.17) | crt (Clostridium acetobutylicum ATCC 824 derived sequence, artificial composition) |
| SEQUENCE NUMBER 4 | vinylacetyl-CoA delta-isomerase (EC number: 5.3.3.3) + 4-hydroxybutyryl-CoA dehydratase (EC number: 4.2.1.120) | provided by the inventors (artificial composition) |
| SEQUENCE NUMBER 5 | acyl-CoA reductase (EC number: 1.2.1.10) | bld (Clostridium saccahroperbutylacetonicum ATCC27021 derived sequence, artificial composition) |
| SEQUENCE NUMBER 6 | acyl-CoA reductase (EC number: 1.2.1.10) | ald (Clostridium beijerinckii NRRL B592 derived sequence, artificial composition) |

### (Comparative example 1)

A blunt end fragment was prepared by an ordinary method in which sequences corresponding to 15 base pairs at an upstream side and at a downstream side including upstream side CAT and downstream side ATG of an NdeI site in multi-cloning sites of an expression vector pET17b (produced by Novagen, Inc.), respectively, were added to an upstream and a downstream, at a 5'-end side and a 3'-end side, of a gene sequence indicated by sequence number 1 (corresponding to β-ketothiolase). Ligation of the fragment and a fragment obtained by performing an NdeI process on pET17b (produced by Novagen, Inc.) was executed by In-Fusion HD Cloning Kit (produced by TAKARA BIO INC.) to obtain plasmid pETBD1.

A gene sequence indicated by sequence number 2 (corresponding to 3-hydroxybutyryl-CoA dehydrogenase) was inserted into pET17b targeting at an NdeI site by a method similar to that of pETBD1 to obtain plasmid pETBD2 containing sequence 2.

An EcoRI site derived from multi-cloning sites of pET17b that was positioned at a downstream of a termination codon of sequence 1 of pETBD1 was cleaved by an enzyme process to prepare a ring-opened fragment of pETBD1. Next, a fragment was prepared by PCR in which sequences corresponding to upstream side 15bp and downstream side 15bp including the EcoRI site of the pETBD1 were added at an upstream and a downstream of a region of the sequence 2 and its upstream region including T7 promoter derived from pET17b of the pETBD2. Ligation of two obtained fragments was executed by an In-Fusion HD Cloning Kit to obtain plasmid pETBD1-2 containing sequences 1 and 2.

Hereinafter, similarly, sequence number 3 (corresponding to enoyl-CoA hydratase), sequence number 4 (corresponding to vinylacetyl-CoA delta-isomerase, 4-hydroxybutyryl CoA dehydratase) and sequence number 5 (corresponding to acyl-CoA reductase) were added in this order to obtain plasmid pETBD1-2-3-4-5. Here, when adding the sequences, opening of plasmid to which sequences were inserted was executed, by cleavage using a restriction enzyme that does not cleave the inserted when an appropriate restriction enzyme site for the restriction enzyme existed on a vector, or by an inverse PCR from a site to be inserted when such a site did not exist. With this, Escherichia coli pETBD1-2-3-4-5/JM109 (DE3) that was transformed from an Escherichia coli JM109 (DE3) stock was obtained.

### (Example 1)

By a method similar to that of Comparative example 1, Escherichia coli pETBD1-2-3-4-6/JM109 (DE3) was obtained that was transformed by plasmid pETBD1-2-3-4-6 in which the gene of sequence 5 of plasmid pETBD1-2-3-4-5 was substituted by a gene indicated by sequence number 6 that codes acyl-CoA reductase of different derivation.

Each of the obtained transformed bodies was aerobically cultured in 5 mL of an LB medium that contained 100 mg/L of ampicillin at 37 °C for 12 hours. 0.1 mL of a culture solution was implanted into 5 mL of an LB medium that contained 1% of glucose, 100 mg/L of ampicillin, and 0.2 mM of IPTG and aerobically cultured at 30 °C for 48 hours. A supernatant of the culture solution was subjected to high performance liquid chromatography (HPLC: column; Shodex SH-1011 (produced by Showa Denko K. K.), column temperature: 60 °C, eluent: 25 mM sulfuric acid aqueous solution, flow rate 0.6 mL/min, detection: differential refraction detector). Table 2 illustrates a relationship between genes that compose plasmids of the used transformed body and the amount of 1,4-butanediol generated in a culturing solution.

**[Table 2]**

| | | COMPARATIVE EXAMPLE 1 | EXAMPLE 1 |
|---|---|---|---|
| TRANSFORMATION GENE (SEQUENCE NUMBER) | *β*-ketothiolase (EC number: 2.3.1.9) | 1 | 1 |
| | 3-hydroxybutyryl-CoA dehydrogenase (EC number: 1.1.1.35) | 2 | 2 |
| | enoyl-CoA hydratase (EC number: 4.2.1.17) | 3 | 3 |
| | vinylacetyl-CoA delta-isomerase (EC number: 5.3.3.3) + 4-hydroxybutyryl-CoA dehydratase (EC number: 4.2.1.120) | 4 | 4 |
| | acyl-CoA reductase (EC number: 1.2.1.10) | 5 | 6 |
| Yield of 1,3-butanediol (mg/L) | | 998 | 970 |
| Yield of 1,4-butanediol (mg/L) | | 36 | 68 |
| Yield ratio of butanediol (1,4-butanediol/1,3-butanediol) | | 0.036 | 0.070 |

As is clearly understood from Table 2, for Example 1 and Comparative example 1, the structures are completely the same except acyl-CoA reductases and it can be considered that the components are formed completely the same until the state of equilibrium between 3-hydroxybutyryl-CoA and 4-hydroxybutyryl CoA. This means that acyl-CoA reductase derived from the gene used in Example 1 is more 4-hydroxybutyryl CoA selective, and with this, it can be understood that the ratio of yield of 1,4-butanediol is improved.

The present application is based on and claims the benefit of priority of Japanese Priority Application No. 2012-257882 filed on November 26, 2012, the entire contents of which are hereby incorporated by reference.

## Claims

1. A method of manufacturing 1,4-butanediol, using a microbe and/or a culture thereof, by an enzyme reaction system that uses acyl-CoA reductase, via 3-hydroxybutyryl-CoA, crotonyl-CoA and 4-hydroxybutyryl CoA in this order,
wherein the reactivity of the acyl-CoA reductase to 4-hydroxybutyryl CoA is greater than or equal to 0.05 times of the reactivity of the acyl-CoA reductase to 3-hydroxybutyryl-CoA.

2. The method of manufacturing 1,4-butanediol according to claim 1,
wherein the acyl-CoA reductase is aldehyde dehydrogenase (acylation)(EC number: 1.2.1.10).

3. The method of manufacturing 1,4-butanediol according to claim 1,
wherein the microbe includes a gene that codes the acyl-CoA reductase and is any one of undermentioned genes (a) to (c):
(a) a gene that has a base sequence of sequence number 6;
(b) a gene that has a base sequence such that one or more bases are deleted, substituted, or added in a base sequence of sequence number 6, wherein the gene has a base sequence with an identity greater than or equal to 90% with respect to the base sequence of sequence number 6;
(c) a gene that hybridizes with a gene that has a base sequence complementary with a gene that has a base sequence described in sequence number 6 on a stringent condition.

4. The method of manufacturing 1,4-butanediol according to claim 1,
wherein the microbe is Escherichia coli, a yeast, a corynebacterium or a clostridial bacteria.

5. A microbe that manufactures 1,4-butanediol by an enzyme reaction system that uses acyl-CoA reductase, via 3-hydroxybutyryl-CoA, crotonyl-CoA and 4-hydroxybutyryl CoA in this order,
wherein the reactivity of the acyl-CoA reductase to 4-hydroxybutyryl CoA is greater than or equal to 0.05 times of the reactivity of the acyl-CoA reductase to 3-hydroxybutyryl-CoA.

6. The microbe according to claim 5,
wherein the microbe is Escherichia coli, a yeast, a corynebacterium or a clostridial bacteria.


<110>   Showa Denko K.K.
<120>   Method of producing 1,4-butanediol and microorganism
<130>   P20120220 (SHD13010PCT)
<160>   6
<170>   PatentIn version 3.5

<210>   1
<211>   1185
<212>   DNA
<213>   Artificial Sequence
<220>
<223>   Escherichia coli atoB gene

<400>   1
atgaaaaatt gtgtcatcgt cagtgcggta cgtactgcta tcggtagttt taacggttca
60

ctcgcttcca ccagcgccat cgacctgggg gcgacagtaa ttaaagccgc cattgaacgt
120

gcaaaaatcg attcacaaca cgttgatgaa gtgattatgg gtaacgtgtt acaagccggg
180

ctggggcaaa atccggcgcg tcaggcactg ttaaaaagcg ggctggcaga aacggtgtgc
240

ggattcacgg tcaataaagt atgtggttcg ggtcttaaaa gtgtggcgct tgccgcccag
300

gccattcagg caggtcaggc gcagagcatt gtggcggggg gtatggaaaa tatgagttta
360

gccccctact tactcgatgc aaaagcacgc tctggttatc gtcttggaga cggacaggtt
420

tatgacgtaa tcctgcgcga tggcctgatg tgcgccaccc atggttatca tatggggatt
480

accgccgaaa acgtggctaa agagtacgga attacccgtg aaatgcagga tgaactggcg
540

ctacattcac agcgtaaagc ggcagccgca attgagtccg gtgcttttac agccgaaatc
600

gtcccggtaa atgttgtcac tcgaaagaaa accttcgtct tcagtcaaga cgaattcccg
660

aaagcgaatt caacggctga agcgttaggt gcattgcgcc cggccttcga taaagcagga
720

acagtcaccg ctgggaacgc gtctggtatt aacgacggtg ctgccgctct ggtgattatg
780

gaagaatctg cggcgctggc agcaggcctt acccccctgg ctcgcattaa aagttatgcc
840

agcggtggcg tgccccccgc attgatgggt atggggccag tacctgccac gcaaaaagcg
900

ttacaactgg cggggctgca actggcggat attgatctca ttgaggctaa tgaagcattt
960

gctgcacagt tccttgccgt tgggaaaaac ctgggctttg attctgagaa agtgaatgtc

1020

aacggcgggg ccatcgcgct cgggcatcct atcggtgcca gtggtgctcg tattctggtc
1080

acactattac atgccatgca ggcacgcgat aaaacgctgg ggctggcaac actgtgcatt
1140

ggcggcggtc agggaattgc gatggtgatt gaacggttga attaa                    1185

<210>  2
<211>  849
<212>  DNA
<213>  Artificial Sequence
<220>
<223>  Clostridium kluyveri hbd gene
<400>  2
atgagtatta aaagtgtagc ggttttaggt agtggaacta tgtctcgtgg aattgtgcag
60

gcttttgcag aagcaggtat agatgtaatt atccgtggaa gaactgaagg tagtattgga
120

aaaggtctag cagcagtaaa gaaagcttat gataaaaaag tatcaaaggg gaaaatttcc
180

caggaagatg ctgataaaat agttggaaga gtaagtacaa caactgaact tgaaaaattg
240

gctgattgtg atcttataat agaagcagca tcagaggata tgaatataaa gaaagactat
300

tttggaaaat tagaagaaat atgcaagcct gaaacaattt ttgctactaa tacttcttca
360

ttatctataa ctgaagtagc aacagctaca aagagaccag ataaattcat aggaatgcat
420

ttctttaatc cagcaaatgt tatgaaatta gttgaaatca taagaggtat gaatacttca
480

caagaaactt ttgatattat aaaagaagct tccattaaaa taggaaaaac tcctgtagaa
540

gttgcagaag ctccaggatt tgttgtaaac aagatattag taccaatgat caatgaagca
600

gtaggaattt tggcagaagg aatagcttca gcagaagata tcgatacagc tatgaaatta
660

ggcgctaatc acccaatggg tcctttagca ttaggagatc ttattggact tgatgtagtt
720

cttgcagtta tggatgtact ttatagtgaa actggagatt caaaatatag agctcataca
780

ttacttagaa aatatgtaag agcaggatgg cttggaagaa aatcaggaaa aggattcttc
840

gcttattaa                                                            849

<210>  3

<211>  786
<212>  DNA
<213>  Artificial Sequence
<220>
<223>  Clostridium acetobutylicum crt gene
<400>  3
atggaactaa acaatgtcat ccttgaaaag gaaggtaaag ttgctgtagt taccattaac
60

agacctaaag cattaaatgc gttaaatagt gatacactaa aagaaatgga ttatgttata
120

ggtgaaattg aaaatgatag cgaagtactt gcagtaattt taactggagc aggagaaaaa
180

tcatttgtag caggagcaga tatttctgag atgaaggaaa tgaataccat tgaaggtaga
240

aaattcggga tacttggaaa taaagtgttt agaagattag aacttcttga aaagcctgta
300

atagcagctg ttaatggttt tgctttagga ggcggatgcg aaatagctat gtcttgtgat
360

ataagaatag cttcaagcaa cgcaagattt ggtcaaccag aagtaggtct cggaataaca
420

cctggttttg gtggtacaca aagactttca agattagttg gaatgggcat ggcaaagcag
480

cttatattta ctgcacaaaa tataaaggca gatgaagcat taagaatcgg acttgtaaat
540

aaggtagtag aacctagtga attaatgaat acagcaaaag aaattgcaaa caaaattgtg
600

agcaatgctc cagtagctgt taagttaagc aaacaggcta ttaatagagg aatgcagtgt
660

gatattgata ctgctttagc atttgaatca gaagcatttg gagaatgctt ttcaacagag
720

gatcaaaagg atgcaatgac agctttcata gagaaaagaa aaattgaagg cttcaaaaat
780

agatag                                                            786


<210>  4
<211>  1473
<212>  DNA
<213>  Artificial Sequence
<220>
<223>  putative gene encoding enzyme converting crotonyl-CoA to
       4-hydroxybutyryl-CoA
<400>  4
atgctgatga ccgccgaaca gtacatcgaa agcctgcgca agctgaacac gcgtgtctac
60

atgtttggtg aaaagatcga gaactgggta gaccatccca tgattcgccc ttcgatcaat
120

tgtgtggcaa tgacctatga actggcgcaa gatccccagt atgccgattt gatgaccacg

180

aaatcgaatc tgattggcaa gacgattaat cgcttcgcga acttacacca aagcaccgat
240

gatctgcgca agaaagtcaa aatgcaacgt ctgctgggcc agaaaaccgc gagttgcttt
300

cagcggtgtg ttgggatgga tgcgttcaat gccgtgtttt ccaccactta tgagattgat
360

cagaaatacg ggactaacta ccacaagaac ttcacggagt atctgaaata tattcaagaa
420

aatgacttga tcgtggatgg tgctatgacc gacccgaaag gcgaccgtgg gctggcaccg
480

agtgcccaga aagacccaga tctgtttctg cgcatcgtgg agaaacgcga ggatggtatc
540

gtagtccgtg gtgcgaaagc acatcaaacg ggctcaatta atagccatga gcacatcatt
600

atgccgacca ttgctatgac agaagcggat aaagactatg cggttagttt tgcttgtcca
660

tccgatgcgg atggtttgtt tatgatctat ggtcgccagt cttgcgacac ccgcaaaatg
720

gaggaaggcg cagatattga cttaggcaac aaacagtttg gcggccaaga agcccttgtc
780

gtgtttgaca acgtattcat cccgaatgat cgcattttct tatgccagga atacgatttt
840

gcgggaatga tggtcgaacg cttcgctggt tatcatcgcc agtcttacgg tggctgcaaq
900

gtgggtgtgg gtgacgtcgt tattggagcc gcagcccttg cagcggatta caacggtgcc
960

cagaaggcca gccatgtgaa agacaaactg atcgaaatga cacacttgaa cgaaaccctg
1020

tattgttgtg ggattgcgtg ctcagccgaa ggttatccga ctgcagccgg caattaccag
1080

atcgatctcc tccttgcgaa tgtgtgcaaa cagaacatta cgcgttttcc gtacgagatt
1140

gtacgtctgg cggaagatat tgccggagga ctgatggtta ctatgcctag cgaagcggac
1200

ttcaaaagcg aaacggttgt tgggcgtgat ggcgaaacca ttggcgattt ctgcaacaaa
1260

ttctttgcgg cagcaccgac ttgcaccaca gaagaacgga tgcgtgtgct ccgctttctg
1320

gagaacattt gtttaggcgc atccgctgtt gggtatcgta cagaatcgat gcatggtgcg
1380

ggctctccac aggctcaacg catcatgatt gcccgtcaag gcaatattaa cgcgaagaaa

1440

gaactcgcta aagcgatcgc tggcatcaaa taa                                1473

<210>  5
<211>  1407
<212>  DNA
<213>  Artificial Sequence
<220>
<223>  Clostridium saccharoperbutylacetonicum bld gene
<400>  5
atgattaaag acacgctagt ttctataaca aaagatttaa aattaaaaac aaatgttgaa
60

aatgccaatc taaagaacta caaggatgat tcttcatgtt tcggagtttt cgaaaatgtt
120

gaaaatgcta taagcaatgc cgtacacgca caaaagatat tatcccttca ttatacaaaa
180

gaacaaagag aaaaaatcat aactgagata agaaaggccg cattagaaaa taaagagatt
240

ctagctacaa tgattcttga agaaacacat atgggaagat atgaagataa aatattaaag
300

catgaattag tagctaaata cactcctggg acagaagatt taactactac tgcttggtca
360

ggagataacg ggcttacagt tgtagaaatg tctccatatg gcgttatagg tgcaataact
420

ccttctacga atccaactga aactgtaata tgtaatagta taggcatgat agctgctgga
480

aatactgtgg tatttaacgg acatccaggc gctaaaaaat gtgttgcttt tgctgtcgaa
540

atgataaata aagctattat ttcatgtggt ggtcctgaga atttagtaac aactataaaa
600

aatccaacta tggactctct agatgcaatt attaagcacc cttcaataaa actactttgc
660

ggaactggag ggccaggaat ggtaaaaacc ctcttaaatt ctggtaagaa agctataggt
720

gctggtgctg gaaatccacc agttattgta gatgatactg ctgatataga aaaggctggt
780

aagagtatca ttgaaggctg ttcttttgat aataatttac cttgtattgc agaaaaagaa
840

gtatttgttt ttgagaacgt tgcagatgat ttaatatcta acatgctaaa aaataatgct
900

gtaattataa atgaagatca agtatcaaag ttaatagatt tagtattaca aaaaaataat
960

gaaactcaag aatactctat aaataagaaa tgggtcggaa aagatgcaaa attattctta
1020

gatgaaatag atgttgagtc tccttcaagt gttaaatgca taatctgcga agtaagtgca
1080

aggcatccat ttgttatgac agaactcatg atgccaatat taccaattgt aagagttaaa
1140

gatatagatg aagctattga atatgcaaaa atagcagaac aaaatagaaa acatagtgcc
1200

tatatttatt caaaaaatat agacaaccta aataggtttg aaagagaaat cgatactact
1260

atctttgtaa agaatgctaa atcttttgcc ggtgttggtt atgaagcaga aggctttaca
1320

actttcacta ttgctggatc cactggtgaa ggaataactt ctgcaagaaa ttttacaaga
1380

caaagaagat gtgtactcgc cggttaa                                    1407

<210>  6
<211>  1407
<212>  DNA
<213>  Artificial Sequence
<220>
<223>  Clostridium beijerinckii ald gene
<400>  6
atgaataaag acacactaat acctacaact aaagatttaa aagtaaaaac aaatggtgaa
60

aacattaatt taaagaacta caaggataat tcttcatgtt tcggagtatt cgaaaatgtt
120

gaaaatgcta taagcagcgc tgtacacgca caaaagatat tatcccttca ttatacaaaa
180

gagcaaagag aaaaaatcat aactgagata agaaaggccg cattacaaaa taaagaggtc
240

ttggctacaa tgattctaga agaaacacat atgggaagat atgaggataa aatattaaaa
300

catgaattgg tagctaaata tactcctggt acagaagatt taactactac tgcttggtca
360

ggtgataatg gtcttacagt tgtagaaatg tctccatatg gtgttatagg tgcaataact
420

ccttctacga atccaactga aactgtaata tgtaatagca taggcatgat agctgctgga
480

aatgctgtag tatttaacgg acacccatgc gctaaaaaat gtgttgcctt tgctgttgaa
540

atgataaata aggcaattat ttcatgtggc ggtcctgaaa atctagtaac aactataaaa
600

aatccaacta tggagtctct agatgcaatt attaagcatc cttcaataaa acttctttgc
660

ggaactgggg gtccaggaat ggtaaaaacc ctcttaaatt ctggtaagaa agctataggt
720

gctggtgctg gaaatccacc agttattgta gatgatactg ctgatataga aaaggctggt
780

aggagcatca ttgaaggctg ttcttttgat aataatttac cttgtattgc agaaaaagaa
840

gtatttgttt ttgagaatgt tgcagatgat ttaatatcta acatgctaaa aaataatgct
900

gtaattataa atgaagatca agtatcaaaa ttaatagatt tagtattaca aaaaaataat
960

gaaactcaag aatactttat aaacaaaaaa tgggtaggaa aagatgcaaa attattctta
1020

gatgaaatag atgttgagtc tccttcaaat gttaaatgca taatctgcga agtaaatgca
1080

aatcatccat ttgttatgac agaactcatg atgccaatat tgccaattgt aagagttaaa
1140

gatatagatg aagctattaa atatgcaaag atagcagaac aaaatagaaa acatagtgcc
1200

tatatttatt ctaaaaatat agacaaccta aatagatttg aaagagaaat agatactact
1260

atttttgtaa agaatgctaa atcttttgct ggtgttggtt atgaagcaga aggatttaca
1320

actttcacta ttgctggatc tactggtgag ggaataacct ctgcaaggaa ttttacaaga
1380

caaagaagat gtgtacttgc cggctaa                                    1407